# EUROPÄISCHE PATENTSCHRIFT

(11) **EP 0 660 702 B1**
(45) Veröffentlichungstag und Bekanntmachung des Hinweises auf die Patenterteilung: **10.12.1997**
(21) Anmeldenummer: 93920705.6
(22) Anmeldetag: 13.09.1993
(51) Int. Cl.: A61K 7/50

(54) **WASSER-IN-ÖL EMULSIONEN, DIE EIN WASSERLÖSLICHES ALKYLGLYCOSID ENTHALTEN**
WATER-IN-OIL EMULSIONS CONTAINING WATER-SOLUBLE ALKYL GLYCOSIDES
EMULSIONS D'EAU EN HUILE CONTENANT UN GLYCOSIDE D'ALKYLE SOLUBLE DANS L'EAU

(30) Priorität: 15.09.1992 DE 4230504
(43) Veröffentlichungstag der Anmeldung: 05.07.1995
(73) Patentinhaber: Beiersdorf Aktiengesellschaft, 20245 Hamburg (DE); HOECHST AKTIENGESELLSCHAFT, 65926 Frankfurt am Main (DE)
(72) Erfinder: KRÖPKE, Rainer, D-21436 Marschacht (DE); PAPE, Wolfgang, D-22041 Hamburg (DE); SCHNEIDER, Günther, D-20251 Hamburg (DE); STAHL, Wilhelm, D-65929 Frankfurt (DE); WIESNER, Matthias, D-55122 Mainz (DE)
(86) Internationale Anmeldenummer: EP9302465
(87) Internationale Veröffentlichungsnummer: WO9406408

(56) Entgegenhaltungen:
- EP-A- 0 458 600
- WO-A-92/02594

## Beschreibung

Die vorliegende Erfindung betrifft stabile kosmetische Mittel, insbesondere Wasser-in-Öl-Emulsionen (W/O-Emulsionen), welche gegen physikalische Zersetzung geschützt sind.

Unter Hautpflege im Sinne der Erfindung ist in erster Linie zu verstehen, daß die natürliche Funktion der Haut als Barriere gegen Umwelteinflüsse (z.B. Schmutz, Fremdstoffe, Mikroorganismen) und gegen den Verlust von körpereigenen Stoffen (z.B. Wasser, Elektrolyte, natürliche feuchtigkeitsbindende Stoffe) gestärkt, unterstützt und, falls nötig, wiederhergestellt wird.

Wird die natürliche Funktion der Haut gestört, kann es zu verstärkter Resorption toxischer und/oder allergener Fremdstoffe oder zum Befall mit pathogenen Mikroorganismen und als Folge zu entzündlichen oder allergischen Hautreaktionen kommen.

Die menschliche Haut verliert durch körpereigene Mechanismen, ebenso wie durch die Transpiration ständig eine gewisse Menge an Feuchtigkeit. Aber auch durch äußere Einflüsse wie die tägliche Körperwäsche, Wind und Wetter verliert die Haut wichtige funktionale Bestandteile. Obwohl die gesunde Haut durchaus imstande ist, diesen Verlust auszugleichen, ist es Ziel der Hautpflege, die Haut beim Ausgleich dieses Verlustes zu unterstützen. Gerade dann aber, wenn das natürliche Regenerationsvermögen, zum Beispiel infolge starker Belastung oder gar einer Erkrankung, nicht ausreicht, ist die Regulation der Hautfeuchtigkeit bzw. anderer relevanter Inhaltsstoffe unerläßlich.

Ziel der Hautpflege ist die also Wiederherstellung und Förderung eines normalen Funktionsbildes der Haut und der Hautanhangsgebilde.

Folgende Methoden der Hautpflege sind an sich bekannt:
a) Okklusion der Haut Wird die Haut mit einem Lipid- oder Lipid/Wasser-Film (herkömmliche Salben oder Crèmes) bedeckt, wird die Barrierefunktion der Haut nicht direkt wiederhergestellt. Der Lipidfilm stellt eine äußere physikalische Schutzschicht dar
b) Behandlung der Haut mit essentiellen Fettsäuren Essentielle Fettsäuren werden derzeit gelegentlich in dermatologischen Präparaten zur Behandlung von trockener Haut eingesetzt.
c) Behandlung der Haut mit keratolytisch wirksamen Substanzen (z.B. Harnstoff, Salicylsäure usw.). Diese Substanzen wirken je nach Art und verwendeter Konzentration keratolytisch, proteolytisch, penetrationsfördernd, epidermisverdünnend, juckreizstillend oder auch wasserbindend. Ihre Verwendung ist im wesentlichen auf medizinische Indikationen beschränkt.
d) Behandlung mit feuchtigkeitsregulierenden Substanzen (z.B. Glycerin, Sorbit usw.) Die Gruppe dieser Substanzen ist von besonderem Interesse, da sie viele gut wirksame Vertreter enthält. Nachteilig ist indes, daß die meisten dieser Stoffe entweder kostspielig oder aber nur unter aufwendigen Bedingungen in kosmetische Zubereitungen einzuarbeiten sind. Zudem gilt für manche Vertreter dieser Gruppe das unter Punkt c) Gesagte.

Zu den hautpflegenden kosmetischen Formulierungen zählen insbesondere die kosmetischen Emulsionen, namentlich die W/O-Emulsionen.

Emulsionen dieser Art können einesteils wirkstofffrei eingesetzt werden, wobei im allgemeinen bereits gute Wirkung erzielt wird, andererseits setzen sich wirkstoffhaltige Emulsionen mehr und mehr durch. Es war also eine Aufgabe der vorliegenden Erfindung, W/O-Emulsionen zur Verfügung zu stellen, welche sowohl als Vehikel für Wirkstoffe der verschiedensten Arten fungieren können, als auch wirkstofffrei über hervorragende dermatologische Eigenschaften verfügen.

Nachteilig an vielen Wasser-in-Öl-Emulsionen des Standes der Technik ist ferner, daß sie nicht stabil gegen physikalische Zersetzung sind. Üblicherweise bezeichnet man das am häufigsten auftretende unerwünschte Verhalten als "Ausölen". Dies besagt, daß sich Wasserphase und Ölphase allmählich trennen. Dies äußert sich beispielsweise darin, daß aus einer W/O-Crème (= Wasser-in-Öl-Crème) Öltropfen austreten. Beginnt aber eine Emulsion erst einmal, sich zu zersetzen, so ist dieser Vorgang nicht mit einfachen Mitteln wieder rückgängig zu machen. Zumindest stehen dem Verbraucher solcher Mittel nicht zur Verfügung.

Besonders empfindlich sind W/O-Emulsionen gegenüber einem Gehalt an stark oberflächenaktiven Stoffen, deren Wasserlöslichkeit hoch ist, im Gegensatz zu Emulgatoren, deren Fettlöslichkeit hoch ist.
Durch Zusatz von stark oberflächenaktiven Stoffen des Standes der Technik wurden W/O-Emulsionen bisher stets zerstört, was sich in Phasentrennung äußerte.

WO-A-92 02 594 offenbart Alkylglycoside als Emulgatoren für Bohrspülungssysteme. In EP-A-0 458 600 werden kosmetische Emulsionen mit Acylglycosiden als Emulgatoren beschrieben.

Aufgabe der vorliegenden Erfindung war es also insgesamt, die Nachteile des Standes der Technik zu beseitigen. Insbesondere sollten stabile hautpflegende W/O-Emulsionen zur Verfügung gestellt werden.

Es hat sich überraschenderweise gezeigt, und darin liegt die Lösung der Aufgabe, daß die Verwendung eines oder mehrerer wasserlöslicher Alkylglycoside zur Herstellung hautpflegender kosmetischer und/oder dermatologischer W/O-Emulsionen den Nachteilen des Standes der Technik abhilft.

Die erfindungsgemäß verwendeten Alkylglycoside zeichnen sich durch die Struktur

Glyc-O-R

aus, bei welcher R, das Aglycon, einen verzweigten oder unverzweigten Alkylrest mit 5 - 18 Kohlenstoffatomen, vorzugsweise einen unverzweigten Alkylrest mit 8 - 10 Kohlenstoffatomen, kennzeichnet.

Glyc- kennzeichnet erfindungsgemäß einen monosaccharidischen Zuckerrest, welcher glycosidisch mit dem Aglycon R verknüpft ist.

Vorteilhafte Verkörperungen der vorliegenden Erfindungen liegen dann vor, wenn Glyc- aus der Gruppe der Hexosylreste gewählt wird, also z.B. Glucosyl-, Mannosyl-, Galactosyl- und Fructosyl-.

Erfindungswesentlich ist in allen Fällen, daß die gewählten Alkylglycoside gut wasserlöslich sind.

Bevorzugt wird die vorliegende Erfindung verkörpert durch die Verwendung eines oder mehrerer wasserlöslicher Alkylglycoside zur Herstellung hautpflegender kosmetischer und/oder dermatologischer W/O-Emulsionen, welche ein Alkylglucosid oder ein Gemisch mehrerer Alkylglucoside enthalten, also solcher Glycoside, in welchen Glyc- einen Glucosylrest kennzeichnet.

Alkylglucoside zeichnen sich durch die Struktur 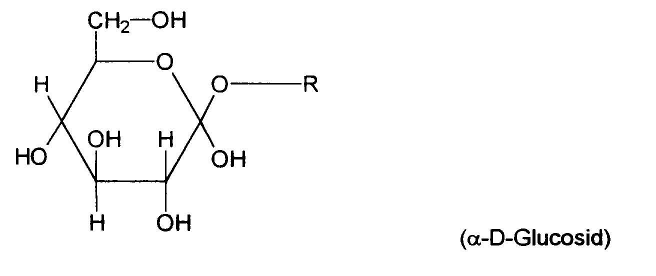 beziehungsweise 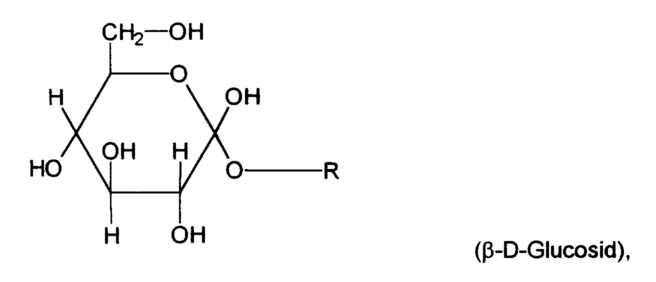 aus. R kennzeichnet einen verzweigten oder unverzweigten Alkylrest mit 5 - 14 Kohlenstoffatomen, vorzugsweise einen unverzweigten Alkylrest mit 8 - 10 Kohlenstoffatomen.

Obwohl die erfindungsgemäß verwendeten Alkylglucoside an sich sowohl aus α-D-Glucosiden wie aus β-D-Glucosiden vorteilhaft gewährt werden können, werden die β-D-Glucoside (bzw. deren spiegelbildliche Formen, die aber weniger leicht zugänglich sind) bevorzugt. Dies beruht darauf, daß die α-D-Glucoside im allgemeinen schlechter wasserlöslich sind als die β-D-Glucoside.

Besondes vorteilhaft zeichnen sich die Alkylglucoside durch folgende Strukturen aus:

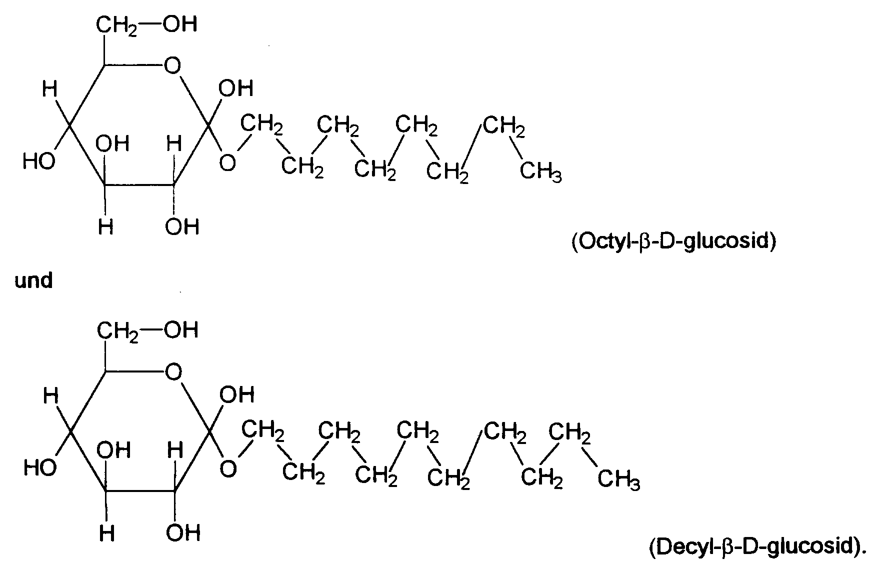

Bevorzugt enthalten die erfindungsgemäß erhältlichen Emulsionen bis zu 0,5 Gew.-% an Alkylglycosiden, vorteilhaft Alkylglucosiden, bevorzugt bis zu 0,3 Gew.-%, insbesondere bis zu 0,3 Gew.-%, jeweils bezogen auf das Gesamtgewicht der W/O-Emulsion. Ganz besonders bevorzugt werden Konzentrationen von 0,001 bis 0,20 Gew.-%, bezogen auf das Gesamtgewicht der W/O-Emulsion, eingesetzt.

Insbesondere ist auch vorteilhaft, die Konzentration der erfindungsgemäßen Alkylglycoside so zu wählen, daß sie kleiner ist als die jeweilige kritische Micellbildungskonzentration (CMC).

Da die Alkylglycoside zu den stark oberflächenaktiven Stoffen zählen, war erstaunlich, daß ein Zusatz von Alkylglycosiden zu W/O-Emulsionen
- stabil sein würde,
- zu kosmetisch besonders anspruchsvollen Emulsionen führen würde,
- zu besonders geschmeidigen Emulsionen führen würde,
- zu Emulsionen mit besonders gut ausgeprägter hautpflegender Wirkung führen,
- zu Vehikeln für mannigfaltige Wirkstoffe führen würde.

Die erfindungsgemäß erhältlichen W/O-Emulsionen enthalten obligatorisch einen W/O-Emulgator bzw. ein Emulgatorgemisch, welches auch ohne Zusatz der erfindungsgemäß verwendeten Alkylglycoside zu W/O-Emulsionen führen würde.

Ohne Alkylglycoside werden aber W/O-Emulsionen enthalten, deren Stabilität weniger groß ist, deren kosmetische Eleganz zu wünschen übrig läßt, welche eher zäh denn geschmeidig sind und deren hautpflegende Wirkung weniger gut ausgeprägt ist. Ferner sind solche W/O-Emulsionen weniger gut als Vehikel für Wirkstoffe geeignet.

Die Alkylglycoside verbessern also die Eigenschaften der W/O-Emulsion, sind aber selbst keine W/O-Emulgatoren.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, antimikrobiell, proteolytisch oder keratolytisch wirksame Substanzen usw.

Entsprechend können die erfindungsgemäßen Zusammensetzungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäß erhältlichen W/O-Emulsionen eigen sich als hautpflegende kosmetische und/oder dermatologische Mittel. Vorteilhaft werden die erfindungsgemäßen W/O-Emulsionen hergestellt, indem das oder die Alkylglycoside sowie die anderen wasserlöslichen Komponenenten in die Wasserphase eingebracht werden, die öllöslichen Komponenten in die Fettphase eingebracht werden und die Wasserphase und die Ölphase bei einer Temperatur, bei welcher beide Phasen flüssig vorliegen, miteinander vereinigt und sodann homogenisiert werden.

Die folgenden Beispiele sollen die Erfindung näher erläutern, ohne daß aber beabsichtigt ist, die Erfindung auf diese Beispiele zu beschränken. Vielmehr ist der Fachmann aufgrund seines Fachwissens in der Lage, Modifikationen vorzunehmen, die den Bereich der vorliegenden Erfindung nicht verlassen.

Die Mengenangaben in den Beispielen beziehen sich auf Gewichts-%, bezogen auf die Gesamtzusammensetzung.

### Beispiel 1

| | Gew.-% |
|---|---|
| Polyglyceryl-3-diisostearat | 2,50 |
| Paraffinwachs | 3,00 |
| Paraffinöl DAB 9 | 10,00 |
| Cetearyloctanoat | 10,00 |
| Bienenwachs | 4,00 |
| Glycerin | 5,00 |
| Decyl-β-D-Glucosid | 0,01 |
| Parfum, Konservierungsmittel, Zusatzstoffe | q.s. |
| Wasser | ad 100,00 |

### Beispiel 2

| | Gew.-% |
|---|---|
| Cholesterin | 1,50 |
| Paraffinwachs | 3,00 |
| Vaseline | 5,00 |
| Paraffinöl DAB 9 | 20,00 |
| Glycerin | 5,00 |
| Decyl-β-D-Glucosid | 0,01 |
| Parfum, Konservierungsmittel, Zusatzstoffe | q.s. |
| Wasser | ad 100,00 |

### Beispiel 3

| | Gew.-% |
|---|---|
| Wollwachsalkohole | 2,50 |
| Paraffinwachs | 6,00 |
| Bienenwachs | 1,00 |
| Vaseline | 3,00 |
| Paraffinöl DAB 9 | 20,00 |
| Glycerin | 5,00 |
| Decyl-β-D-Glucosid | 0,01 |
| Parfum, Konservierungsmittel, Zusatzstoffe | q.s. |
| Wasser | ad 100,00 |

### Beispiel 4

| | Gew.-% |
|---|---|
| Polyglyceryl-3-diisostearat | 2,50 |
| Paraffinwachs | 3,00 |
| Paraffinöl DAB 9 | 10,00 |
| Cetearyloctanoat | 10,00 |
| Bienenwachs | 4,00 |
| Glycerin | 5,00 |
| Octyl-β-D-Glucosid | 0,05 |
| Parfum, Konservierungsmittel, Zusatzstoffe | q.s. |
| Wasser | ad 100,00 |

### Beispiel 5

| | Gew.-% |
|---|---|
| Cholesterin | 1,50 |
| Paraffinwachs | 3,00 |
| Vaseline | 5,00 |
| Paraffinöl DAB 9 | 20,00 |
| Glycerin | 5,00 |
| Octyl-β-D-Glucosid | 0,05 |
| Parfum, Konservierungsmittel, Zusatzstoffe | q.s. |
| Wasser | ad 100,00 |

### Beispiel 6

| | Gew.-% |
|---|---|
| Wollwachsalkohole | 2,50 |
| Paraffinwachs | 6,00 |
| Bienenwachs | 1,00 |
| Vaseline | 3,00 |
| Paraffinöl DAB 9 | 20,00 |
| Glycerin | 5,00 |
| Decyl-β-D-Glucosid | 0,05 |
| Parfum, Konservierungsmittel, Zusatzstoffe | q.s. |
| Wasser | ad 100,00 |

### Beispiel 7

| | Gew.-% |
|---|---|
| Polyglyceryl-3-diisostearat | 1,50 |
| PEG-40-Sorbitanheptaisostearat | 1,50 |
| Paraffinöl DAB 9 | 15,00 |
| Vaseline | 8,00 |
| Paraffinwachs | 6,00 |
| 2-Octyldodecanol | 8,00 |
| Aluminiumstearat | 0,20 |
| Glycerin | 5,00 |
| Decyl-β-D-Glucosid | 0,01 |
| Parfum, Konservierungsmittel, Zusatzstoffe | q.s. |
| Wasser | ad 100,00 |

### Beispiel 8

| | Gew.-% |
|---|---|
| Polyglyceryl-3-diisostearat | 1,50 |
| PEG-40-Sorbitanheptaisostearat | 1,50 |
| Paraffinöl DAB 9 | 15,00 |
| Vaseline | 8,00 |
| Paraffinwachs | 6,00 |
| 2-Octyldodecanol | 8,00 |
| Aluminiumstearat | 0,20 |
| Glycerin | 5,00 |
| Octyl-β-D-Glucosid | 0,05 |
| Parfum, Konservierungsmittel, Zusatzstoffe | q.s. |
| Wasser | ad 100,00 |

### Beispiel 9

| | Gew.-% |
|---|---|
| Polyglyceryl-3-diisostearat | 2,50 |
| Paraffinwachs | 3,00 |
| Paraffinöl DAB 9 | 10,00 |
| Cetearyloctanoat | 10,00 |
| Bienenwachs | 4,00 |
| Glycerin | 5,00 |
| Decyl-β-D-Glucosid | 0,003 |
| Parfum, Konservierungsmittel, Zusatzstoffe | q.s. |
| Wasser | ad 100,00 |

### Beispiel 10

| | Gew.-% |
|---|---|
| Cholesterin | 1,50 |
| Paraffinwachs | 3,00 |
| Vaseline | 5,00 |
| Paraffinöl DAB 9 | 20,00 |
| Glycerin | 5,00 |
| Decyl-β-D-Glucosid | 0,003 |
| Parfum, Konservierungsmittel, Zusatzstoffe | q.s. |
| Wasser | ad 100,00 |

### Beispiel 11

| | Gew.-% |
|---|---|
| Wollwachsalkohole | 2,50 |
| Paraffinwachs | 6,00 |
| Bienenwachs | 1,00 |
| Vaseline | 3,00 |
| Paraffinöl DAB 9 | 20,00 |
| Glycerin | 5,00 |
| Decyl-β-D-Glucosid | 0,003 |
| Parfum, Konservierungsmittel, Zusatzstoffe | q.s. |
| Wasser | ad 100,00 |

### Beispiel 12

| | Gew.-% |
|---|---|
| Polyglyceryl-3-diisostearat | 2,50 |
| Paraffinwachs | 3,00 |
| Paraffinöl DAB 9 | 10,00 |
| Cetearyloctanoat | 10,00 |
| Bienenwachs | 4,00 |
| Glycerin | 5,00 |
| Octyl-β-D-Glucosid | 0,005 |
| Parfum, Konservierungsmittel, Zusatzstoffe | q.s. |
| Wasser | ad 100,00 |

### Beispiel 13

| | Gew.-% |
|---|---|
| Cholesterin | 1,50 |
| Paraffinwachs | 3,00 |
| Vaseline | 5,00 |
| Paraffinöl DAB 9 | 20,00 |
| Glycerin | 5,00 |
| Octyl-β-D-Glucosid | 0,005 |
| Parfum, Konservierungsmittel, Zusatzstoffe | q.s. |
| Wasser | ad 100,00 |

### Beispiel 14

| | Gew.-% |
|---|---|
| Wollwachsalkohole | 2,50 |
| Paraffinwachs | 6,00 |
| Bienenwachs | 1,00 |
| Vaseline | 3,00 |
| Paraffinöl DAB 9 | 20,00 |
| Glycerin | 5,00 |
| Decyl-β-D-Glucosid | 0,003 |
| Parfum, Konservierungsmittel, Zusatzstoffe | q.s. |
| Wasser | ad 100,00 |

### Beispiel 15

| | Gew.-% |
|---|---|
| Polyglyceryl-3-diisostearat | 1,50 |
| PEG-40-Sorbitanheptaisostearat | 1,50 |
| Paraffinöl DAB 9 | 15,00 |
| Vaseline | 8,00 |
| Paraffinwachs | 6,00 |
| 2-Octyldodecanol | 8,00 |
| Aluminiumstearat | 0,20 |
| Glycerin | 5,00 |
| Decyl-β-D-Glucosid | 0,003 |
| Parfum, Konservierungsmittel, Zusatzstoffe | q.s. |
| Wasser | ad 100,00 |

### Beispiel 16

| | Gew.-% |
|---|---|
| Polyglyceryl-3-diisostearat | 1,50 |
| PEG-40-Sorbitanheptaisostearat | 1,50 |
| Paraffinöl DAB 9 | 15,00 |
| Vaseline | 8,00 |
| Paraffinwachs | 6,00 |
| 2-Octyldodecanol | 8,00 |
| Aluminiumstearat | 0,20 |
| Glycerin | 5,00 |
| Octyl-β-D-Glucosid | 0,005 |
| Parfum, Konservierungsmittel, Zusatzstoffe | q.s. |
| Wasser | ad 100,00 |

### Vergleichsversuch 1:

Eine W/O-Emulsion mit der Zusammensetzung gemäß Beispiel 11 wurde hergestellt, indem
Fettphase: aus Wollwachsalkoholen, Paraffinwachs, Bienenwachs, Vaseline und Paraffinöl DAB 9, Temperatur: 75° C
Wasserphase: Glycerin, Decyl-β-D-Glucosid, Wasser (ohne Parfum, Konservierungsmittel, Zusatzstoffe), Temperatur: 75° C
zusammengegeben wurden. Es bildete sich sofort eine geschmeidige Emulsion.

### Vergleichsversuch 2:

Eine W/O-Emulsion gemäß Vergleichsversuch 1 wurde hergestellt, mit dem einzigen Unterschied, daß kein Decyl-β-Glucosid zugegeben wurde. Es dauerte zehn Minuten, bis eine zähe Emulsion gebildet wurde.

### Vergleichsversuch 3:

Eine W/O-Emulsion mit der Zusammensetzung gemäß Beispiel 11 wurde hergestellt, indem
Fettphase: aus Wollwachsalkoholen, Paraffinwachs, Bienenwachs, Vaseline und Paraffinöl DAB 9, Temperatur: 75° C
Wasserphase: Glycerin, Decyl-β-D-Glucosid, Wasser (ohne Parfum, Konservierungsmittel, Zusatzstoffe), Temperatur: 25° C
zusammengegeben wurden. Es bildete sich praktisch sofort eine geschmeidige Emulsion.

### Vergleichsversuch 4:

Eine W/O-Emulsion gemäß Vergleichsversuch 3 wurde hergestellt, mit dem einzigen Unterschied, daß kein Decyl-β-Glucosid zugegeben wurde. Es dauerte etwa zwanzig Minuten, bis eine Emulsion erhalten werden konnte, welche instabil und kosmetisch nicht akzeptabel war.

## Patentansprüche

1. Verwendung eines oder mehrerer wasserlöslicher Alkylglycoside zur Herstellung hautpflegender kosmetischer und/oder dermatologischer W/O-Emulsionen.

2. Verwendung von W/O-Emulsionen, enthaltend ein wasserlösliches Alkylglycosid oder ein Gemisch mehrerer wasserlöslicher Alkylglycoside, als hautpflegende kosmetische und/oder dermatologische Zubereitungen, wobei die Verwendung zur chirurgischen oder therapeutischen Behandlung des menschlichen oder tierischen Körpers und Diagnostizierverfahren, die am menschlichen oder tierischen Körper vorgenommen werden, ausgeschlossen sind.

3. Verwendung nach Anspruch 1 oder 2, dadurch gekennzeichnet, daß das wasserlösliche Alkylglycosid oder die wasserlöslichen Alkylglycoside gewählt werden aus der Gruppe der Alkylglucoside.

4. Verwendung nach Anspruch 3, dadurch gekennzeichnet, daß das oder die Alkylglucoside aus den folgenden Strukturen gewählt werden: 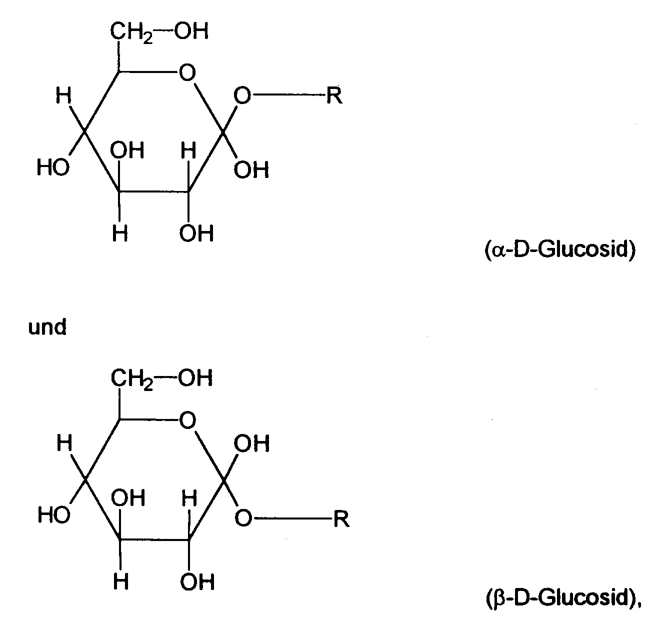 wobei R einen verzweigten oder unverzweigten Alkylrest mit 5 - 18 Kohlenstoffatomen, vorzugsweise einen unverzweigten Alkylrest mit 8 - 10 Kohlenstoffatomen, darstellt.

5. Verwendung nach Anspruch 3, dadurch gekennzeichnet, daß die Alkylglucoside aus der Gruppe der β-D-Glucoside gewählt werden.

6. Verwendung nach Anspruch 3, dadurch gekennzeichnet, daß das oder die Alkylglucoside gewählt werden aus der Gruppe

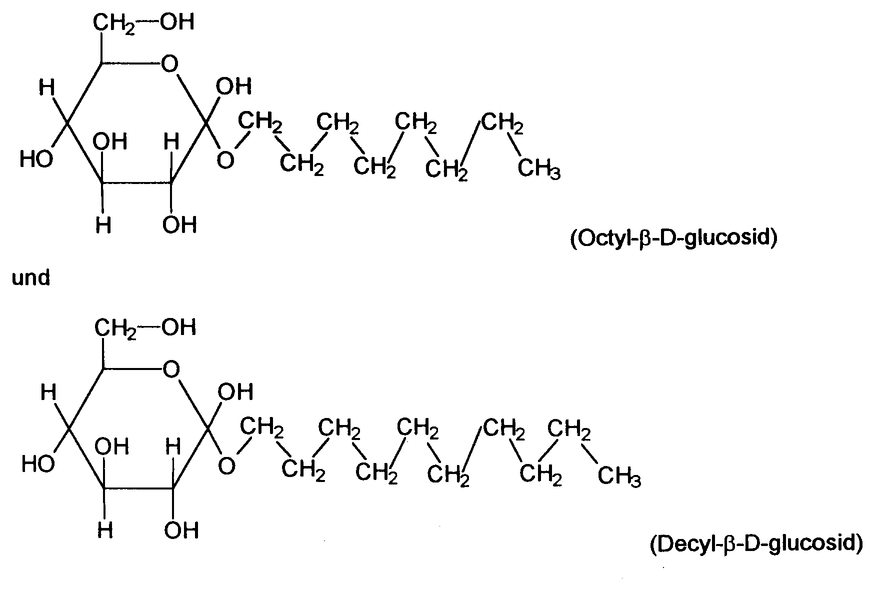

7. Verwendung nach Anspruch 1 oder 2, dadurch gekennzeichnet, daß sie bis zu 1,0 Gew.-%, bevorzugt bis zu 0,5 Gew.-%, insbesondere bis zu 0,3 Gew.-% an Alkylglycosiden, jeweils bezogen auf das Gesamtgewicht der W/O-Emulsion, enthalten.

8. Verwendung nach Anspruch 1 oder 2, dadurch gekennzeichnet, daß das oder die Alkylglycoside in Konzentrationen von 0,001 bis 0,20 Gew.-%, bezogen auf das Gesamtgewicht der W/O-Emulsion, vorliegen.

9. Verfahren zur Erniedrigung der Viskosität von W/O-Emulsionen, dadurch gekennzeichnet, daß eine Wasserphase mit einem wirksamen Gehalt an einem oder mehreren wasserlöslichen Alkylglycosiden mit einer Ölphase vereinigt wird, wobei die Wasserphase und/oder die Ölphase gewünschtenfalls weitere wasser- und/oder öllösliche Bestandteile, darunter Emulgatoren, enthalten können.

## Claims

1. Use of one or more water-soluble alkyl glycosides for preparing cosmetic and/or dermatological skincare W/O emulsions.

2. Use of W/O emulsions comprising a water-soluble alkyl glycoside or a mixture of two or more water-soluble alkyl glycosides as cosmetic and/or dermatological skincare preparations, with the exclusion of use for surgical or therapeutic treatment of the human or animal body and diagnostic techniques practised on the human or animal body.

3. Use according to Claim 1 or 2, characterized in that the water-soluble alkyl glycoside or the water-soluble alkyl glycosides is or are chosen from the group consisting of alkyl glucosides.

4. Use according to Claim 3, characterized in that the alkyl glucoside or alkyl glucosides is or are chosen from the following structures: 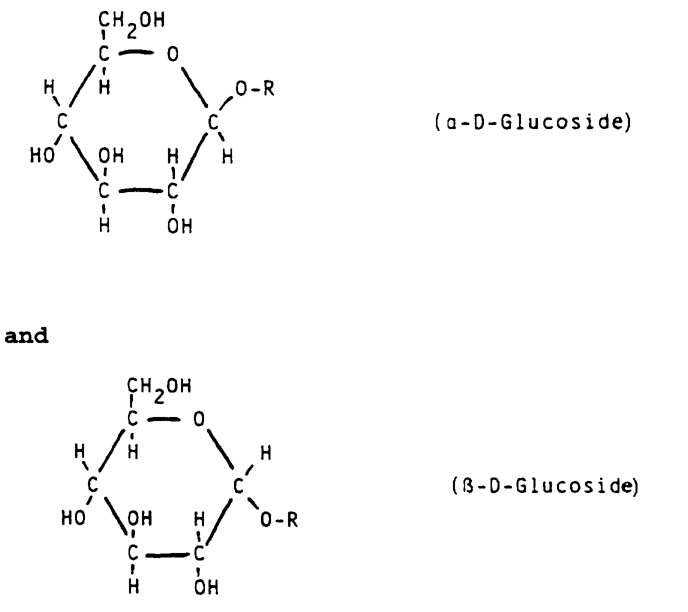 wherein R is a branched or unbranched alkyl radical having 5 - 18 carbon atoms, preferably an unbranched alkyl radical having 8 - 10 carbon atoms.

5. Use according to Claim 3, characterized in that the alkyl glucosides are chosen from the group consisting of β-D-glucosides.

6. Use according to Claim 3, characterized in that the alkyl glucoside or alkyl glucosides is or are chosen from the group consisting of

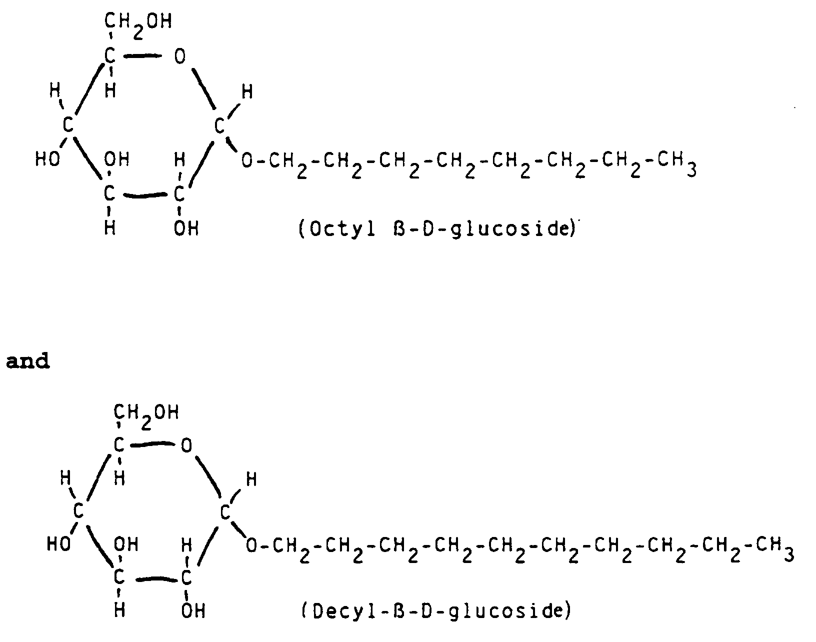

7. Use according to Claim 1 or 2, characterized in that they comprise up to 1.0 % by weight, preferably up to 0.5 % by weight, in particular up to 0.3 % by weight, of alkyl glycosides, in each case based on the total weight of the W/O emulsion.

8. Use according to Claim 1 or 2, characterized in that the alkyl glycoside or alkyl glycosides is or are present in concentrations of 0.001 to 0.20 % by weight, based on the total weight of the W/O emulsion.

9. Process for reducing the viscosity of W/O emulsions, characterized in that an aqueous phase having an active content of one or more water-soluble alkyl glycosides is combined with an oily phase, the aqueous phase and/or the oily phase possibly comprising, if desired, other water- and/or oil-soluble constituents, including emulsifiers.

## Revendications

1. Utilisation d'un ou de plusieurs alkylglycosides hydrodiluables en vue de la fabrication d'émulsions E/H cosmétiques et/ou dermatologiques pour les soins de la peau.

2. Utilisation d'émulsions E/H, contenant un alkylglycoside hydrodiluable ou un mélange de plusieurs alkylglycosides hydrodiluables, en tant que préparations cosmétiques et/ou dermatologiques pour les soins de la peau, l'utilisation en vue d'un traitement chirurgical ou thérapeutique du corps humain ou du corps animal et en vue de procédés diagnostiques, qui sont effectués sur le corps humain ou le corps animal, étant exclue.

3. Utilisation selon la revendication 1 ou 2, caractérisée en ce que l'on choisit l'alkylglycoside hydrodiluable ou les alkylglycosides hydrodiluables parmi le groupe des alkylglucosides.

4. Utilisation selon la revendication 3, caractérisée en ce que l'on choisit l'alkylglycoside ou les alkylglycosides parmi les structures suivantes: 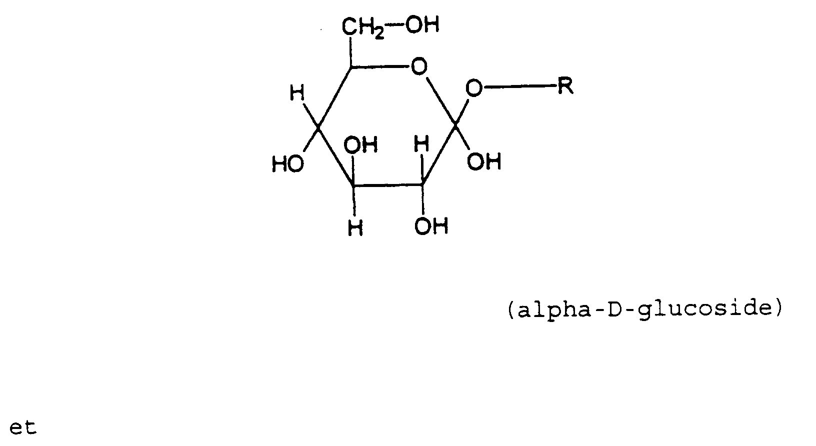 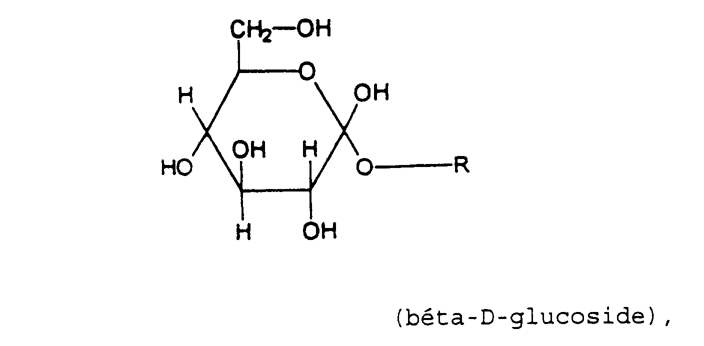 R représentant un résidu alkyle ramifié ou non ramifié ayant 5-18 atomes de carbone, de préférence un résidu non ramifié ayant 8-10 atomes de carbone.

5. Utilisation selon la revendication 3, caractérisée en ce que l'on choisit les alkylglucosides parmi le groupe des béta-D-glucosides.

6. Utilisation selon la revendication 3, caractérisée en ce que l'on choisit l'alkylglucoside ou les alkylglucosides parmi les structures suivantes:

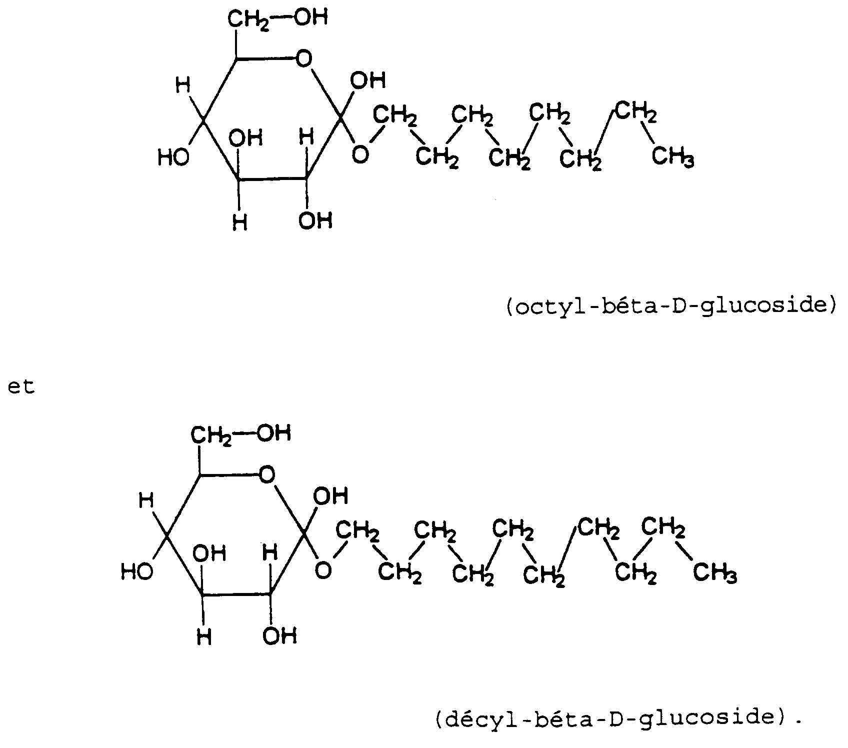

7. Utilisation selon la revendication 1 ou 2, caractérisé en ce qu'ils contiennent, à chaque fois par rapport au poids total de l'émulsion E/H, jusqu'à 1,0 % en poids, de préférence jusqu'à 0,5 % en poids, en particulier jusqu'à 0,3 % en poids d'alkylglycosides.

8. Utilisation selon la revendication 1 ou 2, caractérisée en ce que l'alkylglycoside ou les alkylglycosides sont présents dans des concentrations de 0,001 à 0,20 % en poids, par rapport au poids total de l'émulsion E/H.

9. Procédé de réduction de la viscosité des émulsions E/H, caractérisé en ce qu'une phase aqueuse ayant une teneur active d'un ou de plusieurs alkylglycosides hydrodiluables est jointe à une phase huileuse, la phase aqueuse et/ou la phase huileuse pouvant contenir, si on le souhaite, des constituants hydrosolubles et/ou solubles dans l'huile, supplémentaires, parmi lesquels on compte des émulsifiants.
